(19) Europäisches Patentamt
European Patent Office
Office européen des brevets

(11) Veröffentlichungsnummer: **0 417 543 B1**

(12) EUROPÄISCHE PATENTSCHRIFT

(45) Veröffentlichungstag der Patentschrift: **02.11.94**

(51) Int. Cl.5: **C07D 215/48**

(21) Anmeldenummer: **90116547.2**

(22) Anmeldetag: **29.08.90**

# (54) Verfahren zur Herstellung von 3-Methylchinolin-8-carbonsäure.

(30) Priorität: **09.09.89 DE 3930167**

(43) Veröffentlichungstag der Anmeldung:
**20.03.91 Patentblatt 91/12**

(45) Bekanntmachung des Hinweises auf die Patenterteilung:
**02.11.94 Patentblatt 94/44**

(84) Benannte Vertragsstaaten:
**BE CH DE ES FR GB IT LI NL**

(56) Entgegenhaltungen:
**EP-A- 0 282 778**
**EP-A- 0 294 685**
**GB-A- 951 499**

(73) Patentinhaber: **BASF Aktiengesellschaft**
**Carl-Bosch-Strasse 38**
**D-67063 Ludwigshafen (DE)**

(72) Erfinder: **Hagen, Helmut, Dr.**
**Max-Slevogt-Strasse 17 e**
**D-6710 Frankenthal (DE)**
Erfinder: **Dupuis, Jacques, Dr.**
**Bannwasserstrase 37**
**D-6700 Ludwigshafen (DE)**
Erfinder: **Arbogast, Karlheinz**
**Hochgewann 7**
**D-6704 Mutterstadt (DE)**

EP 0 417 543 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft ein neues Verfahren zur Herstellung von 3-Methylchinolin-8-carbonsäure I

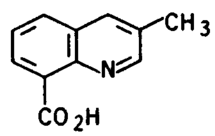

I

und deren Derivaten durch Umsetzung von o-Toluidin II bzw. dessen Derivaten

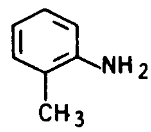

II

in 70-90 gew.-%iger Schwefelsäure in Gegenwart von Jod oder einer Jodverbindung mit Methacrolein III

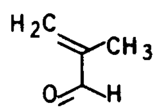

III

und Oxidation des so erhaltenen 3,8-Dimethylchinolins IV bzw. dessen Derivaten

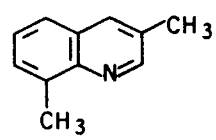

IV

mit Salpetersäure in schwefelsaurer Lösung in Gegenwart von Vanadin-Ionen zur 3-Methylchinolin-8-carbonsäure.

In der EP-A 294 685 wird die Synthese von 3,8-Dimethylchinolin aus o-Toluidin und Methacrolein in Gegenwart von Jod in schwefelsaurer Lösung beschrieben.

Aus der EP-A 282 778 ist die Oxidation von 3,8-Dimethylchinolin und dessen Derivaten zu 3-Methylchinolin-8-carbonsäure und deren Derivaten mittels Salpetersäure in Gegenwart von Vanadin-Ionen in schwefelsaurer Lösung bekannt. Es wird dort jedoch ausdrücklich darauf hingewiesen, daß die Reinheit der Edukte für ein günstiges Ergebnis dieser Umsetzung ausschlaggebend ist.

Die auf diesem Stand der Technik basierende Synthese von 3-Methylchinolin-8-carbonsäure I und deren Derivaten ist jedoch technisch unbefriedigend, da sie die aufwendige Reindarstellung des Zwischenprodukts IV erfordert. Unterläßt man sie und setzt das Reaktionsgemisch der Synthese von IV unmittelbar in die Oxidationsstufe ein, so sinkt die Ausbeute an I. Außerdem wird die Oxidation in diesem Fall von heftigem Schäumen begleitet, welches die Umsetzung verfahrenstechnisch erheblich erschwert.

Die Erfindung beruht auf der Beobachtung, daß die in der ersten Reaktionsstufe bei der Synthese von IV gebildeten Nebenprodukte sowohl für die Minderausbeuten an I als auch für die Schaumbildung bei der Oxidation von IV zu I verantwortlich sind.

Wie festgestellt wurde, handelt es sich bei diesen Nebenprodukten vornehmlich um Polymethacrylate, die unter den Reaktionsbedingungen der Anellierung von II mit III gebildet werden. Ihre Menge beträgt bei Einsatz von 1,4 mol III pro mol II etwa 5 Gew.-% bis 10 Gew.-% des 3,8-Dimethylchinolins IV.

Der vorliegenden Erfindung lag ein einstufiges Verfahren zur Herstellung von 3-Methylchinolin-8-carbonsäure als Aufgabe zugrunde.

Dementsprechend wurde ein Verfahren zur Herstellung von 3-Methylchinolin-8-carbonsäure I

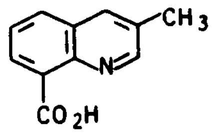

I

und deren Derivaten durch Umsetzung von o-Toluidin II bzw. dessen Derivaten

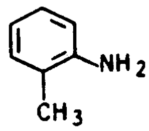

II

in 70-90 gew.-%iger Schwefelsäure in Gegenwart von Jod oder einer Jodverbindung mit Methacrolein III

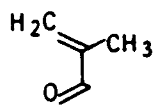

III

oder dessen Derivaten und Oxidation des so erhaltenen 3,8-Dimethylchinolins IV bzw. dessen Derivaten

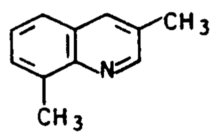

IV

mit Salpetersäure in schwefelsaurer Lösung in Gegenwart von Vanadin-Ionen gefunden, das dadurch gekennzeichnet ist, daß man in der schwefelsauren Reaktionslösung des 3,8-Dimethylchinolins IV zunächst die Reaktionsnebenprodukte oxidativ zersetzt, die so erhaltene 3,8-Dimethylchinolin-Lösung destillativ aufkonzentriert und das 3,8-Dimethylchinolin anschließend in situ mit Salpetersäure zur 3-Methylchinolincarbonsäure oxidiert.

Der Kern des erfindungsgemäßen Verfahrens beruht nun darauf, daß man die schwefelsaure Reaktionslösung oxidativ von Nebenprodukten befreit, so daß eine Isolierung des 3,8-Dimethylchinolins unnötig wird. Daraus ergibt sich, daß sowohl Anellierung als auch Oxidation in einem Medium (in situ) aufeinander folgen können.

Zur oxidativen Zersetzung der Nebenprodukte der Anellierung eignet sich insbesondere 5 bis 65 gew.-%ige wässrige Salpersäure als Oxidationsmittel.

Die Salpetersäure wird zur oxidativen Zersetzung der Reaktionsnebenprodukte in Mengen von 1,2 mol bis 3,0 mol bezogen auf die Verbindung II, vorzugsweise 1,3 bis 2 mol zugesetzt.

Diese Oxidation setzt bei Temperaturen von ca 50 °C ein und verläuft mit hinreichender Geschwindigkeit ab ca 70 °C. Erhitzt man auf über ca. 150 °C setzt wiederum Schaumbildung ein. Optimal arbeitet man hier in einem Temperatur-Bereich von 70 bis 130 °C. Sofern man das Reaktionsgemisch so langsam erhitzt, daß vor Erreichen der maximalen Temperatur die Nebenprodukte bereits teilweise zersetzt sind, kann diese Oxidation auch bei höheren Temperaturen durchgeführt werden

Nach der Zersetzung der Reaktionsnebenprodukte wird die schwefelsaure Lösung von 3,8-Dimethylchinolin durch Abdestillieren von überschüssigem Wasser aufkonzentriert und ohne weitere Vorbehandlung durch Zugabe weiterer Salpetersäure in bekannter Weise oxidiert.

Das erfindungsgemäße Verfahren eignet sich zur Herstellung von 3-Methylchinolin-8-carbonsäure und substituierten 3-Methylchinolin-8-carbonsäuren aus den entsprechenden o-Toluidinen insbesondere solchen der allgemeinen Formel IIa

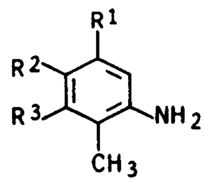

IIa

in welcher die Substituenten $R^1$ bis $R^3$ Wasserstoff, Halogen, C-organische Reste und/oder Nitrogruppen bedeuten, wobei die Natur der Substituenten nach den bisherigen Erkenntnissen auf das Verfahren nur einen geringen Einfluß hat, soweit sie sich unter den Reaktionsbedingungen inert verhalten.

Bevorzugt kommen als Reste $R_1$ bis $R_3$ neben Wasserstoff folgende Gruppen in Betracht:
Halogen wie Fluor, Chlor, Brom und Jod, insbesondere Chlor; Methyl und Aryl wie insbesondere Phenyl.

Diese Reste können ihrerseits durch Heteroatome wie Stickstoff, Sauerstoff und Schwefel unterbrochen sein oder weitere inerte Reste wie Halogen, Nitro, Sulfonyl, Arylsulfonyl und Carboxyl tragen.

Die nach dem erfindungsgemäßen Verfahren besser zugängliche 3-Methylchinolin-8-carbonsäure I und deren Derivate dienen beispielsweise als Pflanzenschutzmittel.

Beispiele

Allgemeine Verfahrensbeispiele zur Herstellung von 3-Methylchinolin-8-carbonsäuren aus o-Toluidinen IIa und Methacrolein III

Variante 1:

a) Anellierung
1 mol o-Toluidinderivat werden gemäß Beispiel 5 der EP-A 294685 mit 1,4 mol Methacrtolein zur Reaktion gebracht.
b) Zerstörung der Nebenprodukte
Eine Lösung aus 2 g (2,2 mol-%) Vanadinpentoxid und 200 g Wasser wurde bei 90 °C innerhalb von 2 Std. Synchron mit 140 g 65 gew.-%iger Salpetersäure und mit der aus 1a) erhaltenen Lösung versetzt.
Nach beendeter Zugabe und weiteren 2 Std. bei 90 °C wurde die Reaktionsmischung auf 155 °C erwärmt, wobei innerhalb von 6 Std. 600 g Wasser abdestilliert wurden.
c) Oxidation zur Chinolincarbonsäure
Diese aufkonzentrierte Reaktionsmischung wurde dann gemäß Beispiel 2 der EP-A 282778 bei 155 °C unter Einleitung von Einleitung von 70 l/Std. Luft innerhalb von 10 Std. mit 450 g 65 gew.-%iger Salpetersäure versetzt. Nach beendeter Oxidation wurde die Reaktionsmischung auf 25 °C abgekühlt und in üblicher weise aufgearbeitet.

Variante 2:

a) Anellierung
Aus einer Reaktionsmischung, die analag zu 1a) aus 1 mol o-Toluidin erhalten wurde, wurden bei 150 °C und 150 mbar 300 ml Wasser abdestilliert.
b) Zerstörung der Polymethacrylate
Eine Lösung aus 2 g (2,2 mol-%) Vanadiumpentoxid und 200 g Wasser wurde bei 90 °C innerhalb von 2 Std. Synchron mit 140 g 65 gew.-%iger Salpetersäure und mit der aus 2a) erhaltenen Lösung versetzt. Nach beendeter Zugabe wurde die Reaktionsmischung weitere 2 Std. bei 90 °C belassen. Die Temperatur wurde dann auf 155 °C erhöht und 300 ml Wasser innerhalb von 4 Std. abdestilliert.
c) Oxidation zur Chinolincarbonsäure
Die Reaktionsmischung aus 2b) wurde auf 155 °C erwärmt und analog zu 1c) unter Einleitung von 70 l/Std. Luft innerhalb von 10 Std. mit 450 g 65 gew.-%iger Salpetersäure oxidiert.

Variante 3:

a) 1 mol o-Toluidin wird wie unter 1a) umgesetzt.
b) Zerstörung der Nebenprodukte
200 g Wasser wurden bei 90 °C innerhalb von 2 Std. synchron mit 140 g 65 gew.-%iger Salpetersäure

und mit der aus 3a) erhaltenen Lösung versetzt. Nach weiteren 4 Std. bei 90 °C wurde die Temperatur auf 155 °C erhöht, wobei in 6 Std. 600 g Wasser abdestilliert wurden.
c) Oxidation zur Chinolincarbonsäure
Zur aufkonzentrierten Lösung aus 3b) wurden 2 g (2,2 mol-%) Vanadiumpentoxid hinzugegeben. Bei 155 °C und unter Einleitung von 70 l/Std. Luft oxidiert man dann mit 450 g 65 gew.-%iger Salpetersäure wie unter 1c).

Variante 4:

a) Anellierung
1 mol o-Toluidin wird analog 1a) umgesetzt.
b) Zerstörung der Nebenprodukte
Die aus 4a) erhaltene Lösung wird wie unter 1b) behandelt.
c) Oxidation zur Chinolincarbonsäure
Die in 4b) behandelte Reaktionsmischung wird innerhalb von 20 Std. und unter Einleitung von 70 l/Std. Luft bei 155 °C mit 1000 g 65 gew.-%iger Salpetersäure oxidiert und in der üblichen Weise aufgearbeitet.

Variante 5:

a) Anellierung
Die Anellierung von 1 mol o-Toluidin erfolgt wie unter 1a).
b) Zerstörung der Nebenprodukte
300 g einer Oxidationslösung, die man wie unter beispielsweise 1c) erhielt und 2 g (2,2 mol-%) Vanadiumpentoxid werden auf 155 °C erwärmt. Innerhalb von 4 Std. werden 140 g 65 gew.-%ige Salpetersäure synchron mit der aus 5a) erhaltenen Reaktionsmischung versetzt, wobei 600 g Wasser abdestilliert werden.
c) Oxidation zur Chinolincarbonsäure
Diese gleichzeitig konzentrierte und von Nebenprodukten befreite Lösung aus 5b) wird wie unter 1c) oxidiert.

Die Einzelheiten der durchgeführten Versuche sind in Tabelle 1 zusammengefaßt.

Tabelle 1

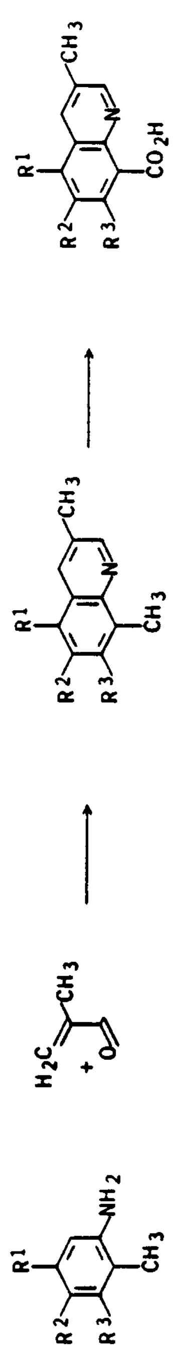

IIa III IVa Ia

| Beispiel Nr. | Edukt IIa R1 | R2 | R3 | Verfahrens-variante | Produkt Ia R1 | R2 | R3 | Ausbeute [%] |
|---|---|---|---|---|---|---|---|---|
| 1 | H | H | Cl | 1 | H | H | Cl | 69 |
| 2 | H | H | Cl | 2 | H | H | Cl | 69 |
| 3 | H | H | Cl | 3 | H | H | Cl | 68 |
| 4 | H | H | Cl | 5 | H | H | Cl | 67 |
| 5 | H | H | $CH_3$ | 4 | H | H | $CO_2H$ | 62 |
| 6 | H | $CH_3$ | H | 4 | H | $CO_2H$ | H | 57 |
| 7 | $CH_3$ | H | H | 1 | $CH_3$ | H | H | 62 |
| 8 | $CH_3$ | H | H | 4 | $CO_2H$ | H | H | 67 |
| 9 | H | $CH_3$ | Cl | 1 | H | $CH_3$ | Cl | 42 |
| 10 | H | $CH_3$ | Cl | 4 | H | $CO_2H$ | Cl | 35 |
| 11 | H | $CO_2H$ | Cl | 1 | H | $CO_2H$ | Cl | 28 |

Der Einfluß der Reaktionsparameter auf die Zerstörung der Nebenprodukte und auf die Ausbeute der darauf folgenden Oxidation zur Chinolincarbonsäure, wurde am Beispiel der Umsetzung von 6-Chlor-o-toluidin IIb mit Methacrolein II zum Chinolin IVb nach folgendem Verfahrensbeispiel untersucht.

Variante 12:

a) Anellierung
1 mol (141,5 g) 6-Chlortoluidin IIb wird gemäß Beispiel 5 EP-A 294685 mit 1,4 mol Methacrolein III versetzt.
b) Zerstörung der Nebenpörodukte
Eine Lösung aus $X_1$ °C innerhalb von $t_1$ Std. Synchron mit $Y_1$ g $Y_2$ gew.-%iger Salpetersäure (1,44 mol) und mit der aus 11a) erhaltene Lösung versetzt.

Nach beendeter Zugabe und Weiteren $t_2$ Std. bei $t_1$ °C wurde die Reaktionsmischung auf 155 °C erwärmt, wobei $X_2$ g Wasser innerhalb von $t_3$ Std. abdestilliert wurden.
c) Oxidation zur 7-Chlor-3-methylchinolin-8-carbonsäure Ib
Die unter 11b) aufkonzentriert Lösung wurde wie bei 1c) oxidiert und aufgearbeitet.

Die Reaktionsparameter für die Zerstörung der Nebenprodukte und die Ausbeuten der anschließenden Oxidation von IVb zur 7-Chglor-3-methylchinolin-8-carbonsäure Ib können Tabelle 2 entnommen werden.

Tabelle 2

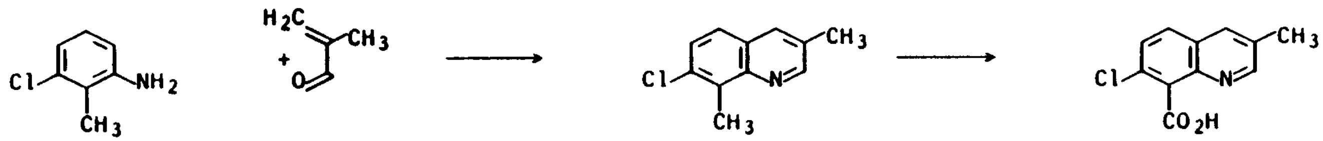

IIb III IVb Ib

| Beispiel Nr. | Menge $V_2O_5X_1$ [g] | Verfahrens-variante | Temperatur $T_1$ [°C] | Gew.-%igkeit der $HNO_3$ $Y_2$ [%] | Menge $HNO_3$ $Y_1$ [g] | Reaktionszeit $t_1+t_2$ [Std.] | Menge Wasser Abzudestill. $X_2$ [g] | Zeit zum Entwässern $t_3$ [Std.] | Ausbeute an Ib bez. auf IIb [%] |
|---|---|---|---|---|---|---|---|---|---|
| 1 | 2 | 1 | 90 | 65 | 140 | 4 | 600 | 6 | 69 |
| 2 | 2 | 2 | 90 | 65 | 140 | 4 | 300 | 4 | 69 |
| 3 | 0 | 3 | 90 | 65 | 140 | 6 | 600 | 6 | 68 |
| 5 | 2 | 5 | 155 | 65 | 140 | 4 | 600 | 0 | 67 |
| 12 | 2 | 12 | 50 | 65 | 140 | 14 | 600 | 7 | 65 |
| 13 | 2 | 12 | 70 | 65 | 140 | 8 | 600 | 7 | 67 |
| 14 | 2 | 12 | 90 | 5 | 1820 | 10 | 2220 | 20 | 68 |
| 15 | 2 | 12 | 90 | 30 | 304 | 6 | 704 | 7 | 69 |
| 16 | 2 | 12 | 110 | 65 | 140 | 4 | 600 | 6 | 68 |

**Patentansprüche**

1. Verfahren zur Herstellung von 3-Methylchinolin-8-carbonsäure I

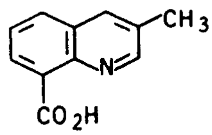

I

und deren Derivaten durch Umsetzung von o-Toluidin II bzw. dessen Derivaten

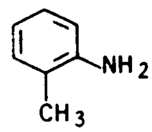

II

in 70-90 gew.-%iger Schwefelsäure in Gegenwart von Jod oder einer Jodverbindung mit Methacrolein III

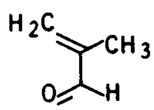

III

und Oxidation des so erhaltenen 3,8-Dimethylchinolins IV bzw. dessen Derivaten

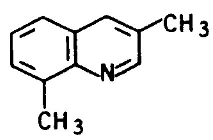

IV

mit Salpetersäure in schwefelsaurer Lösung in Gegenwart von Vanadin-Ionen, dadurch gekennzeichnet, daß man in der schwefelsauren Reaktionslösung des 3,8-Dimethylchinolins IV zunächst die Reaktionsnebenprodukte oxidativ zersetzt, die so erhaltene 3,8-Dimethylchinolin-Lösung destillativ aufkonzentriert und das 3,8-Dimethylchinolin anschließend in situ mit Salpetersäure zur 3-Methylchinolincarbonsäure oxidiert.

2. Verfahren nach Anspruch 1, dadurch gekennzeichnet, daß man für die Zersetzung der Reaktionsnebenprodukte Salpetersäure verwendet.

3. Verfahren nach Anspruch 2, dadurch gekennzeichnet, daß man die Reaktionsnebenprodukte bei 50 bis 150 °C zersetzt.

4. Verfahren nach Anspruch 2, dadurch gekennzeichnet, daß man für die Zersetzung der Reaktionsnebenprodukte 1,2 bis 3,0 mol Salpetersäure pro mol II verwendet.

**Claims**

1. A process for the preparation of 3-methylquinoline-8-carboxylic acid I

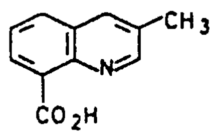

I

or a derivative thereof by reacting o-toluidine II or a derivative thereof

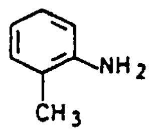

II

with methacrolein III

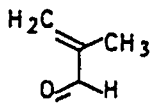

III

in 70-90% strength by weight sulfuric acid in the presence of iodine or of an iodine compound, and oxidizing the resulting 3,8-dimethylquinoline IV or a derivative thereof

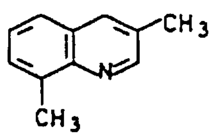

IV

with nitric acid in a solution containing sulfuric acid, in the presence of vanadium ions, wherein, in the sulfuric acid-containing reaction solution of the 3,8-dimethylquinoline IV, the byproducts of the reaction are first decomposed by oxidation, the resulting 3,8-dimethylquinoline solution is concentrated by distillation and the 3,8-dimethylquinoline is then oxidized in situ with nitric acid to give the 3-methylquinolinecarboxylic acid.

**2.** A process as claimed in claim 1, wherein nitric acid is used for decomposing the byproducts of the reaction.

**3.** A process as claimed in claim 2, wherein the byproducts of the reaction are decomposed at from 50 to 150 °C.

**4.** A process as claimed in claim 2, wherein from 1.2 to 3.0 moles of nitric acid per mole of II are used for decomposing the byproducts of the reaction.

**Revendications**

**1.** Procédé de préparation d'acides 3-méthylquinolène-8-carboxyliques

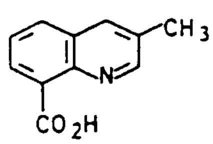

I

et leurs dérivés par réaction de o-toluidine II ou ses dérivés

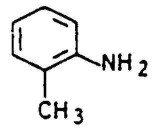

II

dans de l'acide sulfurique à 70-90 % en poids, en présence d'iode ou d'un composé iodé, avec de la méthacroléine III

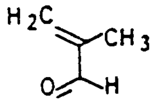

III

et oxydation de la 3,8-diméthylquinoléine IV obtenue ou ses dérivés

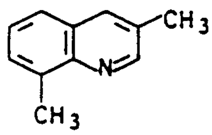

IV

avec de l'acide azotique en solution sulfurique en présence d'ions de vanadine, caractérisé par le fait que dans la solution de réaction sulfurique du 3,8-diméthylquinoléine IV on décompose d'abord par oxydation les produits secondaires de la réaction, on concentre par distillation la solution de 3,8-diéméthylquinoléine ainsi obtenue et on oxyde ensuite in situ la 3,8-diméthylquinoléine avec de l'acide azotique pour obtenir l'acide 3-méthylquinoléinecarboxylique.

**2.** Procédé selon la revendication 1, caractérisé par le fait que l'on utilise l'acide azotique pour la décomposition des produits secondaires de réaction.

**3.** Procédé selon la revendication 2, caractérisé par le fait que l'on décompose les produits secondaires de réaction à 50 à 150 °C.

**4.** Procédé selon la revendication 2, caractérisé par le fait que l'on utilise, pour la décomposition des produits secondaires de réaction, 1,2 à 3,0 moles d'acide azotique par mole II.